# DEMANDE DE BREVET EUROPEEN

(11) **EP 1 438 951 A1**
(43) Date de publication de la demande: **21.07.2004**
(21) Numéro de dépôt: 04290075.3
(22) Date de dépôt: 12.01.2004
(51) Int. Cl.: A61K 7/135

(54) **Compositions de décoloration prêtes à l'emploi, procédé de préparation et procédé de décoloration**

(30) Priorité: 16.01.2003 FR 0300456
(71) Demandeur: L'OREAL, 75008 Paris (FR)
(72) Inventeur: Legrand, Frédéric, 92400 Courbevoie (FR); Kravtchenko, Sylvain, 92600 Asnieres (FR)
(74) Mandataire: Wattremez, Catherine

(57) **Abrégé**

L'invention a pour objet une composition de décoloration de fibres kératiniques humaines, et en particulier des cheveux, prête à l'emploi, susceptible d'être obtenue en mélangeant avant emploi i) une composition de décoloration anhydre sous forme de pâte comprenant au moins un sel peroxygéné, au moins un agent alcalin et de 15 à 35 % d'au moins un liquide inerte organique, avec ii) une composition oxydante sous forme d'une émulsion huile dans eau de peroxyde d'hydrogène, comprenant au moins un tensioactif non ionique et/ou anionique et au moins un copolymère comprenant au moins un motif provenant d'un monomère à insaturation éthylénique à groupement sulfonique, sous forme libre ou partiellement ou totalement neutralisée et comprenant au moins une partie hydrophobe.

Elle a de plus pour objet un procédé de préparation de ladite composition, consistant à mélanger avant emploi la composition de décoloration avec la composition oxydante.

Elle concerne un procédé de décoloration de fibres kératiniques humaines, consistant à appliquer la composition de décoloration prête à l'emploi selon l'invention, à la laisser agir puis à l'éliminer par un rinçage à l'eau, suivi d'un lavage avec un shampooing, puis éventuellement d'un séchage, ainsi qu'un dispositif pour sa mise en oeuvre.

## Description

La présente invention a pour objet une composition de décoloration de fibres kératiniques, un procédé de décoloration de fibres kératiniques la mettant en oeuvre ainsi qu'un dispositif à plusieurs compartiments la comprenant.

Il est connu de décolorer des fibres kératiniques humaines, et en particulier les cheveux, en utilisant des composition de décoloration comprenant un ou plusieurs agents oxydants. Parmi les agents oxydants classiquement utilisés, on peut citer le peroxyde d'hydrogène ou des composés susceptibles de produire du peroxyde d'hydrogène par hydrolyse, comme par exemple l'urée ou les persels tels que les perborates, les persulfates, les percarbonates.
A l'origine, les compositions de décoloration se présentaient sous la forme de poudre. Elles avaient cependant l'inconvénient de produire de la poussière durant leur manutention, leur transport et leur stockage. De plus, ce phénomène était aggravé par le fait que les produits qui composent ces poudres sont corrosifs, irritants pour les yeux, pour les voies respiratoires et les muqueuses. C'est pourquoi, afin de résoudre les problèmes rencontrés avec l'usage de compositions pulvérulentes, on a récemment développé compositions de décoloration sous forme de pâte. Ainsi, les composés pulvérulents sont dispersés dans un support liquide inerte organique épaissi.
Si cette galénique apporte une solution aux problèmes de volatilité évoqués ci-dessus, la mise en oeuvre des compositions sous forme de pâte occasionne de nouvelles difficultés.

Ainsi, les compositions de décoloration, quelles soient sous forme de poudre ou de pâte, sont à mélanger avant emploi, à des compositions aqueuses de peroxyde d'hydrogène pour obtenir la composition de décoloration prête à l'emploi.

Ces compositions aqueuses de peroxyde d'hydrogène, sont sous forme de solutions aqueuses ou d'émulsions huile dans eau et sont plus ou moins liquides ou fluides.
Cette galénique favorise les mélanges avec des compositions de décoloration sous forme de poudre, dans la mesure où plus la composition aqueuse de peroxyde d'hydrogène est liquide ou fluide, plus la poudre décolorante se solubilise rapidement et facilement.
En revanche, les compositions de décoloration sous forme de pâte sont dépourvues d'eau et leur texture est compacte et dure. De plus, ces pâtes décolorantes sont à tendance hydrophobe compte tenu de la présence d'un taux élevé de liquide inerte organique. En conséquence, le mélange de la composition de décoloration et de la composition de peroxyde d'hydrogène est loin d'être facilité. Cela se traduit non seulement par un temps de mélange plus long mais aussi par une complication des opérations pour obtenir un mélange homogène.

L'une des solutions envisagées a été d'enrichir les émulsions huile dans eau de peroxyde d'hydrogène, de corps gras tels des alcools gras, afin d'obtenir des textures crèmes plus compactes. Toutefois, le différentiel de texture par rapport aux pâtes décolorantes anhydres reste important, et les mélanges sont toujours relativement longs à réaliser.

Il convient donc de trouver des compositions aqueuses oxydantes particulières dont le mélange avec des pâtes décolorantes soit plus rapide et plus facile.

La présente invention a pour objet de résoudre les problèmes mentionnés ci-dessus.

Ainsi, elle a pour objet une composition de décoloration de fibres kératiniques humaines, et en particulier des cheveux, prête à l'emploi, susceptible d'être obtenue en mélangeant avant emploi i) une composition de décoloration anhydre sous forme de pâte comprenant au moins un sel peroxygéné, au moins un agent alcalin et de 15 à 35 % d'au moins un liquide inerte organique, avec ii) une composition oxydante sous forme d'une émulsion huile dans eau de peroxyde d'hydrogène, comprenant au moins un tensioactif non ionique et/ou anionique et au moins un copolymère comprenant au moins un motif provenant d'un monomère à insaturation éthylénique à groupement sulfonique, sous forme libre ou partiellement ou totalement neutralisée et comprenant au moins une partie hydrophobe.

Elle a de plus pour objet un procédé de préparation de ladite composition, consistant à mélanger avant emploi ladite composition de décoloration anhydre sous forme de pâte avec ladite composition oxydante sous forme d'une émulsion huile dans eau de peroxyde d'hydrogène.

Un autre objet de l'invention concerne un procédé de décoloration de fibres kératiniques humaines, en particulier des cheveux, consistant à appliquer la composition de décoloration prête à l'emploi selon l'invention, sur la zone des fibres kératiniques humaines, sèches ou humides, à décolorer ; à la laisser agir pendant un temps de pause suffisant pour obtenir la décoloration recherchée ; à éliminer la composition par un rinçage à l'eau, suivi d'un lavage avec un shampooing, puis éventuellement d'un séchage.

Enfin, l'invention concerne un dispositif à plusieurs compartiments, ou "kit", pour la mise en oeuvre du procédé de décoloration précité, comportant au moins deux compartiments dont l'un contient une composition de décoloration anhydre sous forme de pâte comprenant au moins un sel peroxygéné, au moins un agent alcalin et de 15 à 35 % d'au moins un liquide inerte organique ; et l'autre contient une composition oxydante sous la forme d'une émulsion huile dans eau de peroxyde d'hydrogène, comprenant au moins un tensioactif non ionique et/ou anionique et au moins un copolymère provenant d'au moins un motif provenant d'un monomère à insaturation éthylénique à groupement sulfonique, sous forme libre ou partiellement ou totalement neutralisée et comprenant au moins une partie hydrophobe.

Il a été trouvé que les mélanges de composition de décoloration anhydre sous forme de pâte avec des émulsions huile dans eau de peroxyde d'hydrogène comprenant au moins un tensioactif non ionique et/ou anionique et un copolymère tel que décrit ci-dessus, pouvaient être atteints de manière significativement plus rapide, et de manière plus aisée.

De plus, les compositions de décoloration prêtes à l'emploi selon l'invention s'appliquent facilement et rapidement. Elles présentent une très bonne adhérence et ne coulent pas hors des zones que l'on souhaite décolorer.
Elles permettent enfin des décolorations puissantes et homogènes, tout en offrant de très bonnes propriétés cosmétiques.

Mais d'autres caractéristiques et avantages de la présente invention apparaîtront plus clairement à la lecture de la description et des exemples qui vont suivre.

La composition de décoloration anhydre va tout d'abord être décrite.

Dans ce qui va suivre, lorsque des gammes sont définies comme comprises entre deux valeurs, ces valeurs sont incluses.

### Composition de décoloration anhydre

Comme indiqué auparavant, ladite composition anhydre est sous forme de pâte. Plus particulièrement, cette pâte présente une teneur en eau inférieure à 1% en poids, et de préférence inférieure à 0,5% en poids par rapport au poids total de la pâte.

### Sel peroxygénés

Par ailleurs, la composition de décoloration anhydre comprend au moins un sel peroxygéné, qui est plus spécialement choisi parmi les persulfates, perborates, percarbonates, et peroxydes de métaux alcalins ou alcalino-terreux, comme le sodium, le potassium, le magnésium.
De préférence, la pâte comprend, à titre de sels peroxygénés, des persulfates, et préférence des persulfates de sodium et de potassium, seuls ou en mélange.

Habituellement, la teneur en sel peroxygéné dans la composition de décoloration anhydre est comprise entre 10 et 70 % en poids, de préférence entre 20 et 60 % en poids. Il est à noter que de manière avantageuse, la teneur en sel peroxygéné dans la composition prête à l'emploi est comprise entre 5 et 35 % en poids de la composition prête à l'emploi (c'est-à-dire comprenant le mélange de la composition de décoloration et la composition oxydante), de préférence entre 10 et 30 % en poids de la composition prête à l'emploi.

### Agents alcalins

La composition de décoloration anhydre comprend en outre au moins un agent alcalin qui est usuellement choisi parmi l'urée ; les sels d'ammonium, comme les chlorures, les sulfates, les phosphates, les nitrates ; les silicates, les phosphates, les carbonates de métaux alcalins (tels que le sodium, le potassium) ou alcalino-terreux (comme notamment le magnésium) seuls ou combinés.

Habituellement, la teneur en agent alcalin dans la composition de décoloration anhydre est comprise entre 0,01 et 40 % en poids, de préférence entre 0,1 et 30 % en poids. Selon un mode de réalisation particulier de l'invention, la teneur en agent alcalin dans la composition prête à l'emploi est comprise entre 0,005 et 20 % en poids, de préférence entre 0,05 et 15 % en poids.

### Liquide inerte

La composition de décoloration anhydre comprend enfin de 15 à 35 % en poids d'au moins un liquide inerte organique.
Par liquide, on entend un composé ou un mélange de composés liquides à 25°C et à pression atmosphérique.

Avantageusement, le liquide inerte organique est choisi parmi les polydécènes, les mono- et poly- esters d'acides carboxyliques, les mono- ou poly- esters de sucres d'acides en C8-C30, les éthers cycliques, les esters cycliques, les huiles silicones, les huiles minérales, les huiles végétales, ou leurs mélanges.

Plus particulièrement, les polydécènes sont des composés de formule C10nH[(20n)+2] avec n variant de 3 à 9, et de préférence de 3 à 7. Ces composés répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes.
On peut citer, à titre d'exemple, le produit vendu sous la dénomination Silkflo® 366 NF Polydecene par la société Amoco Chemical, ceux vendus sous la dénomination Nexbase@ 2002 FG, 2004 FG, 2006 FG et 2008 FG par la société Fortum.

En ce qui concerne les mono- ou poly- esters d'acides carboxyliques, ces derniers, linéaires ou ramifiés, saturés ou non, comprennent avantageusement au moins une chaîne hydrocarbonée en C8-C30, plus particulièrement en C8-C24, de préférence C12-C24, provenant de la partie acide ou alcool, et au moins une chaîne en C1-C8, de préférence en C1-C6. De plus, si l'acide carboxylique comprend plusieurs fonctions carboxyliques, celles-ci sont de préférence toutes estérifiées. Enfin, il est à noter que les alcools sont de préférence des alcools monofonctionnels.

A titre d'exemples, on peut citer les esters des acides oléique, laurique, palmitique, myristique, béhénique, stéarique, linoléique, linolénique, caprique, arachidonique, ou leurs mélanges comme notamment les mélanges oléo-palmitique, oléo-stéarique, palmito-stéarique, etc.
On peut de plus citer le diester isopropylique de l'acide sébacique (sébaçate de diisopropyle), l'adipate de di-octyle et le maléate de di-caprylyle.

On peut de plus utiliser un polyester d'un acide polycarboxylique comprenant un radical avec moins de 6 atomes de carbone, saturé ou non, linéaire ou ramifié, et d'un alcool comprenant un radical avec moins de 6 atomes de carbone, saturé ou non, linéaire ou ramifié. A titre d'exemple, on peut citer le citrate de triéthyle.

De préférence, les esters sont choisis parmi ceux obtenus à partir d'acides en C12-C24, plus particulièrement comprenant un groupement carboxylique, et d'un monoalcool saturé, linéaire ou ramifié, en C3-C6.
Selon une variante particulièrement avantageuse de l'invention, la composition de décoloration comprend, à titre de liquide inerte, le palmitate d'isopropyle, et de préférence le myristate d'isopropyle, seuls ou en mélanges.

Pour ce qui a trait aux mono- ou poly- esters de sucres d'acides en C8-C30, de préférence en C12-C24, il est précisé que le terme sucre désigne des composés qui possèdent plusieurs fonctions hydroxyle, avec ou sans fonction aldéhyde ou cétone, et qui comportent au moins 4 atomes de carbone. Ces sucres peuvent être des monosaccharides, des oligosaccharides ou des polysaccharides. Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.
Quant aux acides en en C8-C30, saturés ou non, linéaires ou ramifiés, comprenant une ou deux fonctions carboxyliques, on pourra se référer aux listes données auparavant.
Les esters peuvent être choisis parmi les mono-, di-, tri- et tétra-esters, les polyesters et leurs mélanges.
Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.
On peut citer, à titre d'exemples, le produit vendu sous la dénomination Glucate DO par la société Amerchol, qui est un dioléate de méthylglucose, les produits vendus sous les dénominations F160, F140, F110, F90, F70, SL40 par la société Crodesta, désignant respectivement les palmito-stéarates de sucrose formés de 73% de monoester et 27% de di- et tri-ester, de 61% de monoester et 39% de di-, tri-, et tétra-ester, de 52% de monoester et 48% de di-, tri-, et tétra-ester, de 45% de monoester et 55% de di-, tri-, et tétra-ester, de 39% de monoester et 61 % de di-, tri-, et tétra-ester, et le mono-laurate de sucrose ; les produits vendus sous la dénomination Ryoto Sugar Esters par exemple référencés B370 et correspondant au béhénate de saccharose formé de 20% de monoester et 80% de di-triester-polyester ; le mono-di-palmito-stéarate de sucrose commercialisé par la société Goldschmidt sous la dénomination Tegosoft PSE.

En ce qui concerne les ester et éthers cycliques, on peut citer par exemple la γ-butyrolactone, le diméthyl isosorbide (dénomination CTFA), ou le diisopropyl isosorbide (dénomination CTFA).

Concernant les huiles silicones, on utilise en tant que liquide inerte des composés qui sont plus particulièrement liquides et non volatiles, de préférence de viscosité inférieure ou égale à 10 000 mPa.s à 25°C, la viscosité des silicones étant mesurée selon la norme ASTM 445 Appendice C.
Les huiles de silicone sont définies plus en détail dans l'ouvrage de Walter NOLL "Chemistry and Technology of Silicones" (1968) - Academic Press.
Toutefois, parmi les huiles de silicone convenables, on peut citer notamment les huiles de silicones vendues sous les dénominations DC-200 fluid-5 mPa.s, DC-200 fluid-20 mPa.s, DC-200 fluid-350 mPa.s, DC-200 fluid- 1000 mPa.s, DC-200 fluid-10 000 mPa.s par la société Dow Corning.

L'huile de paraffine est un exemple d'huile minérale susceptible d'être utilisée comme liquide inerte de la composition de décoloration anhydre.

En ce qui concerne les huiles végétales, on peut citer tout particulièrement l'huile d'avocat, l'huile d'olive ou la cire liquide de jojoba.

### Additifs

### Polymères amphiphiles :

La pâte de décoloration anhydre peut de plus comprendre les additifs usuels tels que les polymères amphiphiles comportant au moins une chaîne hydrophobe. Plus spécialement ces polymères amphiphiles, s'ils sont présents sont de type non ioniques, anioniques, cationiques ou amphotères. De préférence ils sont de nature non ionique, anionique ou cationique.

Notons que le(s) polymère(s) amphiphile(s) présent(s) dans la composition de décoloration anhydre et celui présent dans la composition oxydante et qui sera détaillé plus loin, sont de préférence différents.

Plus particulièrement, lesdits polymères amphiphiles comprennent, en tant que chaîne hydrophobe, une chaîne hydrocarbonée, saturée ou non, aromatique ou non, linéaire ou ramifiée, en C8-C30, comprenant éventuellement un ou plusieurs motifs oxyalkylénés (oxyéthylénés et/ou oxypropylénés).

Parmi les polymères amphiphiles cationique comportant une chaîne hydrophobe on peut trouver des polyuréthannes cationiques ou des copolymères cationiques comprenant des motifs vinyllactame et en particulier vinylpyrrolidone.
Encore plus préférentiellement les polymères amphiphiles comportant une chaîne hydrophobe seront de nature nonionique ou anionique.

A titre d'exemples de polymères amphiphiles non ioniques à chaîne hydrophobe, on peut citer entre autres :
**(1)** les celluloses modifiées par des groupements comportant au moins une chaîne hydrocarbonée, saturée ou non, linéaire ou ramifiée, en C6-C30, comme les hydroxyéthylcelluloses modifiées par des groupements comportant au moins une chaîne hydrophobe telle que définie auparavant, comme notamment Natrosol Plus Grade 330 CS (alkyles en C16 - commercialisé par la société Aqualon) ; Bermocoll EHM 100 (commercialisé par la société Berol Nobel), Amercell Polymer HM-1500 (hydroxyéthylcellulose modifiée par un groupement polyéthylène glycol (15) éther de nonylphénol - commercialisé par la société Amerchol).
**(2)** les hydroxypropylguars modifiés par des groupements comportant au moins une chaîne hydrophobe telle que définie, par exemple Jaguar XC-95/3 (chaîne alkyle en C₁₄ - commercialisé par la société Rhodia Chimie) ; Esaflor HM 22 (chaîne alkyle en C₂₂ - commercialisé par la société Lamberti) ; RE210-18 (chaîne alkyle en C₁₄) et RE205-1 (chaîne alkyle en C₂₀) commercialisés par la société Rhodia Chimie.
**(3)** les copolymères de vinylpyrrolidone et de monomères hydrophobes à chaîne hydrophobe telle que définie auparavant comme par exemple Antaron ou Ganex V216 (copolymères vinylpyrrolidone / hexadécène) ; Antaron ou Ganex V220 (copolymères vinylpyrrolidone / eicosène), commercialisés par la société I.S.P.
**(4)** les copolymères de (méth)acrylates d'alkyles en C₁-C₆ et de monomères amphiphiles comportant une chaîne hydrophobe.
**(5)** les copolymères de (méth)acrylates hydrophiles et de monomères hydrophobes comportant au moins une chaîne hydrophobe, tels que par exemple le copolymère méthacrylate de polyéthylèneglycol / méthacrylate de lauryle.
**(6)** les polymères à squelette aminoplaste éther possédant au moins une chaîne grasse, tels que les composés Pure Thix commercialisés par la société Süd-Chemie.
**(7)** les polyéthers polyuréthannes, linéaires (structure à blocs), greffés ou en étoile, comportant dans leur chaîne, au moins une séquence hydrophile, généralement polyoxyéthylénée et pouvant comprendre entre 50 et 1000 motifs oxyéthylène environ, et au moins une séquence hydrophobe, qui peut comprendre des groupements aliphatiques seuls, éventuellement combinés à des enchaînements cycloaliphatiques et/ou aromatiques. De préférence, les polyéthers polyuréthanes comportent au moins deux chaînes hydrophobes hydrocarbonées en C6-C30, séparées par une séquence hydrophile ; les chaînes hydrophobes pouvant être des chaînes pendantes ou des chaînes à l'une ou plusieurs des extrémités de la ou des séquences hydrophiles.
   Les polyéthers polyuréthannes comportent une liaison uréthanne entre les séquences hydrophiles, d'où l'origine du nom. Par extension figurent aussi parmi les polyéthers polyuréthannes dont les séquences hydrophiles sont liées aux séquences lipophiles par d'autres liaisons chimiques.
   Les polyéthers polyuréthannes utilisables selon l'invention sont en particulier ceux décrits dans l'article de G. Fonnum, J. Bakke et Fk. Hansen - Colloid Polym. Sci 271, 380.389 (1993). A titre d'exemples de polyéthers polyuréthannes, on peut citer Nuvis FX 1100 (désignation I.N.C.I. européenne et américaine "Steareth-100/PEG-136/H.M.D.l. Copolymer" commercialisé par la société Servo Delden) ; Rhéolate 205, 208 , 204 ou 212 (commercialisés par la société Rheox) ; Elfacos T210 (chaîne alkyle en C₁₂- C₁₄) Elfacos T212 (chaîne alkyle en C₁₈) comemrcialisés par la société Akzo.

Les polymères amphiphiles anioniques à chaîne hydrophobe, susceptibles d'être mis en oeuvre comportent au moins à titre de chaîne hydrophobe, une chaîne hydrocarbonée, saturée ou non, aromatique ou non, linéaire ou ramifiée, en C₈-C₃₀.

Plus particulièrement les polymères amphiphiles anioniques comportant au moins une chaîne hydrophobe utilisables dans le cadre de la présente invention, réticulés ou non, comprennent au moins un motif hydrophile dérivé d'un ou de plusieurs monomères à insaturation éthylénique portant une fonction acide carboxylique, libre ou partiellement ou totalement neutralisée, et au moins un motif hydrophobe dérivé d'un ou de plusieurs monomères à insaturation éthylénique portant une chaîne latérale hydrophobe, et éventuellement au moins un motif de réticulation dérivés d'un ou plusieurs monomères polyinsaturés.

A titre de monomère à insaturation éthylénique portant une fonction acide carboxylique, on peut citer l'acide éthacrylique, l'acide méthacrylique et l'acide acrylique, ou leurs mélanges ; les deux derniers étant préférés.
A titre d'exemples de monomères à insaturation éthylénique portant une chaîne latérale hydrophobe peuvent être cités les esters d'acides carboxyliques insaturés, comme notamment les acides éthacrylique, méthacrylique, acrylique, et d'alcools saturés, linéaires ou ramifiés, en C₁₀-C₃₀, plus particulièrement en C₁₂-C₂₂. Ils peuvent aussi être des éthers allyliques d'alcools saturés ou non, aromatiques ou non, ramifiés ou non, en C₆-C₃₀, éventuellement oxyalkyléné (de préférence oxyéthyléné), plus particulièrement de formule CH₂=CR'CH₂OBₙR, avec R' représentant H ou CH₃, B représentant le radical éthylèneoxy, n est un entier compris entre 0 et 100, R représente un radical hydrocarboné choisi parmi les radicaux alkyle, arylalkyle, aryle, alkylaryle, cycloalkyle, comportant de 8 à 30 atomes de carbone. Un motif plus particulièrement préféré est tel que R' représente l'hydrogène, n est égal à 10, et R représente un radical stéaryle (C₁₈).
Concernant le monomère réticulant, celui-ci comprend au moins deux doubles liaisons polymérisables non conjuguées l'une avec l'autre. On peut citer à titre d'exemples le phtalate de diallyle, le (méth)acrylate d'allyle, le divinylbenzène, le diméthacrylate de (poly)éthylèneglycol, le méthylène-bis-acrylamide, le polyallylsucrose ou le polyallylpentaérythritol.
Des polymères amphiphiles anioniques du type décrit ci-dessus sont décrits et préparés, par exemple dans les brevets US 3915921 et US 4509949 (copolymères d'acide (méth)acrylique et de (méth)acrylates d'alkyles en C10-C30) ou dans le brevet EP 216479 (copolymères d'acide (méth)acrylique et d'éthers allyliques d'alcools gras).

On peut citer à titre d'exemples de polymères préférés, Carbopol ETD-2020 (copolymère acide acrylique/méthacrylate d'alkyle en C10-C30, réticulé - commercialisé par la société Goodrich) ; Carbopol 1382, Pemulen TR1, Pemulen TR2 (copolymères acide acrylique/acrylate d'alkyle en C₁₀₋₃₀, réticulés - commercialisés par la société Goodrich). le copolymère acide méthacrylique/acrylate d'éthyle/méthacrylate de stéaryle oxyéthyléné (55/35/10); le copolymère acide (méth)acrylique/acrylate d'éthyle/méthacrylate de béhényle oxyéthyléné 25 OE, et le copolymère réticulé acide méthacrylique/acrylate d'éthyle/stéareth-10 allyl éther.

Si ces polymères amphiphiles sont présents, leur teneur représente de 0,01 à 30 % en poids de la composition de décoloration prête à l'emploi.

### Polymères hydrosolubles épaississants dépourvus de chaîne hydrophobe :

La composition de décoloration anhydre peut de plus comprendre au moins un polymère hydrosoluble épaississant dépourvu de chaîne hydrophobe.
Les polymères convenables peuvent être d'origine naturelle, ou être des polymères synthétiques, et sont avantageusement choisis parmi ceux utilisés classiquement dans le domaine cosmétique. En outre, ces polymères ne présentent pas de chaîne hydrophobe, c'est-à-dire une chaîne hydrocarbonée, saturée ou non, aromatique ou non, linéaire ou ramifiée, en C8-C30, comprenant éventuellement un ou plusieurs motifs oxyalkylénés (oxyéthylénés et/ou oxypropylénés). Les polymères hydrosolubles épaississants sont donc différents des polymères amphiphiles qui viennent d'être décrits.

Comme exemples de polymères synthétiques, on peut citer, la polyvinylpyrrolidone, l'acide polyacrylique, le polyacrylamide, l'acide poly-2-acrylamidopropanesulfonique non réticulé (Simugel EG de la société SEPPIC), l'acide poly-2-acrylamido-2-méthylpropane sulfonique réticulé, libre ou partiellement neutralisé par l'ammoniaque (Hostacerin AMPS de Clariant), des mélanges d'acide poly-2-acrylamido-2-méthylpropane sulfonique non réticulé avec des éthers d'hydroxyalkylcellulose ou avec des poly(oxyde d'éthylène) tel qu'ils sont décrits dans le brevet US 4540510 ; des mélanges d'acide poly(méth)acrylamido-alkyl(C₁-C₄)-sulfonique, de préférence réticulé, avec un copolymère réticulé de l'anhydride maléique et d'un alkyl(C₁-C₅)vinyléther (Hostacerin AMPS / Stabileze QM de la société ISF).

Les polymères épaississants d'origine naturelle utilisables selon la présente invention, sont de préférence des polymères comportant au moins un motif sucre, comme les gommes de guar non ioniques, modifiées ou non par des groupements hydroxyalkyle en C₁-C₆ ; les gommes de biopolysaccharides d'origine microbienne telles que les gommes de Scléroglucane ou de Xanthane ; les gommes issues d'exudats végétaux telles que les gommes Arabique, Ghatti, Karaya, Tragacanthe, Carraghénanne, Agar et Caroube ; les pectines ; les alginates ; les amidons ; les hydroxyalkyl(C₁-C₆)celluloses et carboxyalkyl(C₁-C₆)celluloses.

Ici, "motif sucre" désigne une portion monosaccharidique (i.e. monosaccharide ou oside ou sucre simple), une portion oligosaccharidique (chaînes courtes formées de l'enchaînement d'unités monosaccharidiques, éventuellement différentes) ou une portion polysaccharidique [longues chaînes constituées d'unités monosaccharidiques, éventuellement différentes, i.e. polyholosides ou polyosides]. Les unités saccharidiques peuvent être en outre substituées par des radicaux alkyle, ou hydroxyalkyle, ou alcoxy, ou acyloxy, ou carboxyle, les radicaux alkyle comportant de 1 à 4 atomes de carbone.

A titre d'exemples de gommes de guar non ioniques non modifiées, on peut citer entre autres Guargel D/15 (Goodrich) ; Vidogum GH 175 (Unipectine), Meypro-Guar 50 et Jaguar C (Meyhall/Rhodia Chimie) ; et à titre de gommes de guar non ioniques modifiées, Jaguar HP8, HP60, HP120, DC 293, HP 105 (Meyhall/Rhodia Chimie) ; Galactasol 4H4FD2 (Aqualon).

Les gommes de biopolysaccharides d'origine microbienne, végétale sont bien connues de l'homme de l'art et décrites notamment dans l'ouvrage de Robert L. Davidson intitulé "Handbook of Water soluble gums and resins" édité chez Mc Graw Hill Book Company (1980).
Parmi ces gommes, citons les scléroglucanes comme notamment Actigum CS de Sanofi Bio Industries ; Amigel de Alban Muller International, ainsi que les scléroglucanes traités au glyoxal décrits dans FR 2633940) ; les gommes xanthanes comme Keltrol, Keltrol T, Keltrol Tf, Keltrol Bt, Keltrol Rd, Keltrol Cg (Nutrasweet Kelco), Rhodicare S, Rhodicare H (Rhodia Chimie) ; les dérivés d'amidon comme Primogel (Avebe) ; les hydroxyéthylcelluloses telles que Cellosize QP3L, QP4400H, QP30000H, HEC30000A, Polymer PCG10 (Amerchol), Natrosol 250HHR, 250MR, 250M, 250HHXR, 250HHX, 250HR, HX (Hercules), Tylose H1000 (Hoechst) ; les hydroxypropylcelluloses comme Klucel EF, H, LHF, MF, G (Aqualon) ; les carboxyméthylcelluloses comme Blanose 7M8/SF, raffinée 7M, 7LF, 7MF, 9M31F, 12M31XP, 12M31P, 9M31XF, 7H, 7M31, 7H3SXF (Aqualon), Aquasorb A500 (Hercules), Ambergum 1221 (Hercules), Cellogen HP810A, HP6HS9 (Montello), Primellose (Avebe).

Les polymères épaississants hydrosolubles dépourvus de chaîne hydrophobe, s'ils sont présents, représentent de 0,01 à 30 % en poids de la composition de décoloration anhydre.

### Tensioactifs :

La composition de décoloration anhydre peut de plus comprendre un ou plusieurs tensioactifs, choisis parmi les tensioactifs anioniques, non ioniques, cationiques, amphotères, zwitterioniques ou leurs mélanges.

A titre d'exemples non limitatifs de tensioactifs anioniques utilisables, seuls ou mélanges, dans le cadre de la présente invention, on peut citer notamment les sels (en particulier sels alcalins, notamment de sodium, magnésium, sels d'ammonium, d'amines, d'aminoalcools) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglycoside citrates, tartrates et sulfosuccinates, les alkylsulfosuccinamates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras (notamment en C6-C24) tels que les sels des acides oléique, ricinoléique, et palmitique, des acides d'huile de coprah ou d'huile de coprah hydrogénée, et notamment de préférence les sels de sodium, calcium ou magnésium de l'acide stéarique; les acyl-lactylates dont le radical acyle comporte 8 à 20 atomes de carbone. On peut également utiliser les acides d'alkyl D galactoside uroniques et leurs sels, les acides alkyl(C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

Sans vouloir s'y limiter, les tensioactifs non ioniques peuvent être choisis, seuls ou en mélange, parmi les alcools, les alpha-diols, les alkylphénols polyéthoxylés et/ou polypropoxylés, ayant une chaîne comportant par exemple 6 à 24 atomes de carbone, le nombre de groupements oxyde d'éthylène et/ou oxyde de propylène pouvant aller notamment de 1 à 50. On peut également citer les copolymères d'oxyde d'éthylène et de propylène, les condensats d'oxyde d'éthylène et de propylène sur des alcools gras (notamment en C₆-C₂₄) ; les amides gras (notamment en C₆-C₂₄) polyéthoxylés ayant de préférence de 2 à 30 moles d'oxyde d'éthylène, les alcools gras (notamment en C₆-C₂₄) mono- ou poly-glycérolés comportant en moyenne 1 à 30 groupements glycérol et les amides gras (notamment en C₆-C₂₄) polyglycérolés comportant en moyenne 1 à 5 groupements glycérol et en particulier 1,5 à 4 ; les esters d'acides gras (notamment en C₆-C₂₄) du sorbitan oxyéthylénés ayant par exemple de 2 à 30 moles d'oxyde d'éthylène ; les esters d'acides gras (notamment en C₆-C₂₄) du sucrose, les esters d'acides gras (notamment en C₆-C₂₄) du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

A titre d'illustration, les agents tensioactifs amphotères ou zwitterioniques peuvent être notamment des dérivés d'amines secondaires ou tertiaires aliphatiques, dans lesquels le radical aliphatique est une chaîne linéaire ou ramifiée, en C₈-C₁₈, et contenant au moins un groupe anionique du type carboxylate, sulfonate, sulfate, phosphate ou phosphonate ; des alkyl(C₈-C₂₀)bétaïnes, les alkyl(C₈-C₂₀)sulfobétaïnes, les alkyl(C₈-C₂₀) amidoalkyl (C₁-C₆)bétaïnes ou les alkyl(C₈-C₂₀)amidoalkyl(C₁-C₆)sulfobétaïnes. Conviennent aussi les ampho- carboxyglycinates les amphocarboxypropionates, classés dans le dictionnaire CTFA, 5ème édition, 1993, notamment sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylampho-dipropionate, Disodium Capryloamphodipropionate, Lauroamphodipropionic acid, Cocoamphodipropionic acid. A titre d'exemples on peut citer le Cocoamphodiacétate (Miranol® C2M concentré de Rhodia Chimie).

Parmi les tensioactifs cationiques on peut citer à titre purement indicatif, les sels d'amines grasses primaires, secondaires ou tertiaires, éventuellement polyoxyalkylénées; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.

De préférence, s'ils sont présents, les tensioactifs sont choisis parmi les composés anioniques et/ou non ioniques.

Au cas où un ou plusieurs tensioactifs seraient présents dans la composition de décoloration anhydre, leur teneur est telle que la teneur totale en tensioactif dans la composition prête à l'emploi représente de 0,05 à 30 %, et de préférence 0,1 à 20 % en poids.

### Polymère substantif cationique ou amphotère :

Selon une variante de l'invention, la composition de décoloration comprend au moins un polymère substantif cationique ou amphotère. Ce type de polymères permet d'améliorer les propriétés cosmétiques des fibres (effet conditionneur).
Rappelons que, au sens de la présente invention, l'expression "polymère cationique" désigne tout polymère contenant des groupements cationiques et/ou des groupements ionisables en groupements cationiques.

Les polymères cationiques ou amphotères convenables sont de manière avantageuse, choisis parmi ceux déjà connus en soi comme améliorant les propriétés cosmétiques des cheveux, à savoir notamment ceux décrits dans les brevets et demandes de brevet EP 337 354, FR 2 270 846, FR 2 383 660, FR 2 598 611, FR 2 470 596, FR 2 519 863, FR 2 788 974, FR 2 788 976 pour avoir une liste de ces composés.

Cependant, à titre d'exemples plus précis de polymères cationiques, on peut notamment citer les polymères cationiques comprenant au moins des groupements amine primaire, secondaire, tertiaire et/ou quaternaire pouvant, soit faire partie de la chaîne principale polymère, soit être portés par un substituant latéral directement relié à celle-ci.

A titre d'exemples plus précis de ces polymères, on peut citer :
**(1)** les copolymères d'acrylamide et de diméthyl-amino-éthyl méthacrylate quaternisé au sulfate de diméthyle ou avec un halogénure de diméthyle (Hercofloc de Hercules) ; les copolymères d'acrylamide et de chlorure de méthacryloyloxy-éthyl-triméthylammonium (Bina Quat P 100 de Ciba Geigy) ; le copolymère d'acrylamide et de méthosulfate de méthacryloyloxy-éthyl-triméthylammonium (Reten de Hercules) ; les copolymères vinylpyrrolidone / acrylate ou méthacrylate de dialkylaminoalkyle quaternisés ou non (gamme Gafquat d'ISP ; Copolymer 845, 958 et 937 de Gaf Corporation (ISP ?)) ; les terpolymères méthacrylate de diméthyl amino éthyle/ vinylcaprolactame/ vinylpyrrolidone (Gaffix VC 713 d'ISP) ; les copolymères vinylpyrrolidone / méthacrylamidopropyl dimethylamine (Styleze CC 10 d'ISP) ; les copolymères vinylpyrrolidone / méthacrylamide de diméthyl-amino-propyle quaternisés (Gafquat HS 100 d'ISP).
**(2)** Les dérivés d'éthers de cellulose comportant des groupements ammonium quaternaire tels que décrits dans FR 1 492 597. Ces polymères sont également définis dans le dictionnaire CTFA comme des ammonium quaternaires d'hydroxyéthylcellulose ayant réagi avec un époxyde substitué par un groupement triméthylammonium.
**(3)** Les dérivés de cellulose cationiques tels que les copolymères de cellulose ou les dérivés de cellulose greffés avec un monomère hydrosoluble d'ammonium quaternaire, et décrits notamment dans US 4 131 576, tels que les hydroxyalkylcelluloses, comme les hydroxyméthyl-, hydroxyéthyl- ou hydroxypropylcelluloses greffées notamment avec un sel de méthacryloyléthyl triméthylammonium, de méthacrylamidopropyltriméthylammonium, de diméthyl-diallylammonium.
**(4)** Les polysaccharides cationiques décrits plus particulièrement dans les brevets US 3 589 578, US 4 031 307, tels que les gommes de guar contenant des groupements cationiques trialkylammonium. On utilise par exemple des gommes de guar modifiées par un sel, comme le chlorure notamment, de 2,3-époxypropyl triméthylammonium.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans FR 2 162 025, FR 2 280 361.
**(6)** Les polyaminoamides solubles dans l'eau préparés en particulier par polycondensation d'un composé acide avec une polyamine, éventuellement réticulés, éventuellement alcoylés ou s'ils comportent une ou plusieurs fonctions amines tertiaires, quaternisées. Ces polymères sont notamment décrits dans FR 2 252 840 et FR 2 368 508.
**(7)** Les dérivés de polyaminoamides résultant de la condensation de polyalkylènes polyamines avec des acides polycarboxyliques suivie d'une alcoylation par des agents bifonctionnels. On peut citer par exemple les polymères acide adipique-dialkylamino hydroxyalkyldialkylène triamine dans lesquels le radical alkyle est en C1-C4. De tels polymères sont notamment décrits dans FR 1 583 363.
**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés en C3-C8, puis avec l'épichlorhydrine. De tels polymères sont notamment décrits dans US 3 227 615, US 2 961 347.
**(9)** Les cyclopolymères d'alkyl diallyl amine ou de dialkyl diallyl ammonium, sous forme d'homopolymère ou de copolymère, tels que décrits dans FR 2 080 759 et dans son certificat d'addition n°2 190 406.
**(10)** Les polymères de diammonium quaternaire tels que décrits dans FR 2 320 330, FR 2 270 846, FR 2 316 271, FR 2 336 434, FR 2 413 907, US 2 273 780, US 2 375 853, US 2 388 614, US 2 454 547, US 3 206 462, US 2 261 002, US 2 271 378, US 3 874 870, US 4 001 432, US 3 929 990, US 3 966 904, US 4 005 193, US 4 025 617, US 4 025 627, US 4 025 653, US 4 026 945, US 4 027 020.
   On peut utiliser plus particulièrement les polymères qui sont constitués de motifs récurrents répondant à la formule suivante: 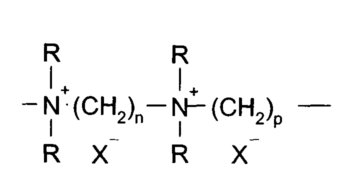 dans laquelle les radicaux R, identiques ou non, désignent un radical alkyle ou hydroxyalkyle en C1-C4, n et p sont des nombres entiers variant de 2 à 20 et, X⁻ est un anion dérivé d'un acide minéral ou organique.
**(11)** Les polymères de poly(ammonium quaternaire) constitués de motifs récurrents de formule : 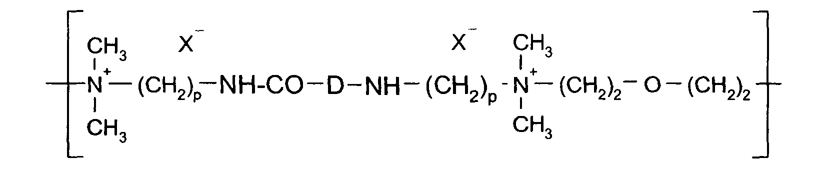 dans laquelle p désigne un nombre entier variant de 1 à 6 environ, D peut être nul ou peut représenter un groupement -(CH₂)ᵣ -CO- dans lequel r désigne un nombre égal à 4 ou à 7, X- est un anion. De tels polymères peuvent être préparés selon les procédés décrits dans US 4 157 388, US 4 702 906, US 4 719 282, EP 122 324.
**(12)** Les polymères quaternaires de vinylpyrrolidone et de vinylimidazole.
**(13)** Les polyamines du type Polyethyleneglycol (15) Tallow Polyamine(dénomination du dictionnaire CTFA).
**(14)** Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale (Salcare® SC 92 de Allied Colloids). On peut également utiliser un homopolymère réticulé du chlorure de méthacryloyloxyéthyl triméthylammonium contenant environ 50 % en poids de l'homopolymère dans de l'huile minérale ou dans un ester liquide (Salcare® SC 95, SC 96 de Allied Colloids).

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

En ce qui concerne les polymères amphotères, on peut utiliser des polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a réagi avec une polyamine comportant un ou plusieurs groupements amine primaire ou secondaire.

Les polymères amphotères répondant à la définition donnée ci-dessus plus particulièrement préférés sont choisis parmi les polymères suivants :
**(1)** Les polymères résultant de la copolymérisation d'un monomère dérivé d'un composé vinylique portant un groupement carboxylique tel que plus particulièrement l'acide (méth)acrylique, l'acide maléique, l'acide alpha-chloracrylique, ou encore un sel de dialkyldiallylammonium tel que le chlorure de diméthyldiallylammonium, et d'un monomère basique dérivé d'un composé vinylique substitué contenant au moins un atome basique tel que plus particulièrement les dialkyl-amino-alkyl-méthacrylate et acrylate, les dialkyl-amino-alkyl-méthacrylamide et acrylamide, comme décrits dans US 3 836 537. On peut citer le copolymère acrylate de sodium / chlorure d'acrylamidopropyl trimethyl ammonium (Polyquart KE 3033 de Henkel), le copolymère acide acrylique/ chlorure de diméthyldiallylammonium (Merquat 280, 295, Plus 3330, de Calgon).
**(2)** Les polymères comportant des motifs dérivant a) d'au moins un monomère choisi parmi les (méth)acrylamides N-substitués par un radical alkyle, notamment en C2-C12 (par exemple éthyl, tertiobutyl, tertiooctyl, octyl, décyl, le dodécyl), b) d'au moins un monomère acide contenant un ou plusieurs groupements carboxyliques réactifs (par exemple acides (méth)acrylique, crotonique, itaconique, et les monoesters des acides ou anhydrides maléique, fumarique), et c) d'au moins un monomère basique tel que des esters à substituant amine primaire, secondaire, tertiaire et quaternaire des acides (méth)acrylique, fumarique, maléique, et le produit de quaternisation du méthacrylate de diméthyl-amino-éthyle avec le sulfate de diméthyle ou diéthyle (par exemple les méthacrylates d'aminoéthyle, de butylaminoéthyle, de N,N'-diméthyl-aminoéthyle, de N-tertio-butyl-aminoéthyle. )
   On utilise particulièrement les copolymères Octylacrylamide / acrylate / butylaminoéthylméthacrylate (Amphomer ou Lovocryl 47 par la société National Starch).
**(3)** Les polyaminoamides réticulés et partiellement ou totalement alcoylés, dérivant de polyaminoamides de formule générale -[CO-R-CO-Z]- dans laquelle R est un radical divalent dérivé d'un acide dicarboxylique saturé ou non (par exemple les acides adipique, triméthyl-2,2,4-adipique et triméthyl-2,4,4-adipique, téréphtalique, itaconique), d'un acide monocarboxylique insaturé (comme l'acide (méth)acrylique), d'un ester d'alcool en C1-C6 des acides précités ou d'un radical dérivant de l'addition de l'un de ces acides avec une amine bis-primaire ou -secondaire, et Z désigne un radical d'une polyalkylène-polyamine bis-primaire, mono ou bis-secondaire. De préférence, Z représente entre 60 et 100 moles %, le radical -NH-[(CH2)x-NH]p- avec x=2 et p=2 ou 3, ou x=3 et p=2 ; ce radical dérivant de la diéthylène triamine, de la triéthylène tétraamine ou de la dipropylène triamine; entre 0 et 40 moles % le radical ci-dessus, dans lequel x=2 et p=1 et qui dérive de l'éthylènediamine, ou le radical dérivant de la pipérazine -N[CH₂CH₂]₂N- ; entre 0 et 20 moles %, le radical -NH-(CH₂)₆-NH- dérivant de l'hexaméthylènediamine. L'agent réticulant de ces polymères est un agent bifonctionnel choisi parmi les épihalohydrines, les diépoxydes, les dianhydrides, les dérivés bis insaturés, et alcoylés par action d'acide acrylique, d'acide chloracétique ou d'une alcane sultone (comme la propane ou la butane sultone) ou de leurs sels de métaux alcalins.
**(4)** Les polymères comportant au moins des motifs zwittérioniques, comme par exemple le copolymère de méthacrylate de butyle / méthacrylate de diméthylcarboxy-méthyl-ammonio-éthyle (Diaformer Z301 de Sandoz).
**(5)** Les polymères dérivés du chitosane comportant des motifs monomères répondant aux formules (I). (II). (III) suivantes : 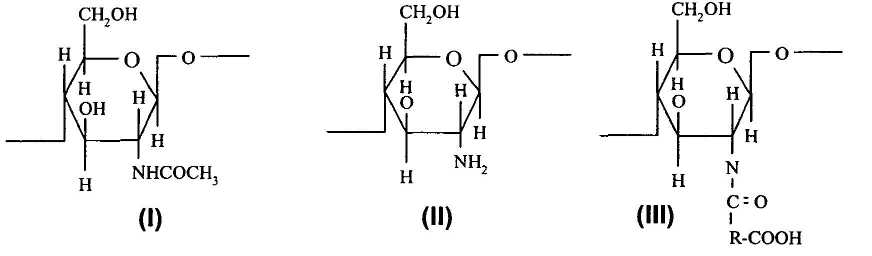 avec (I) représentant de 0 à 30%, (II) de 5 à 50% et (III) de 30 à 90% dans lequel R représente un radical de formule : 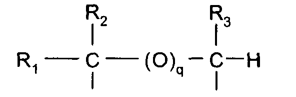 dans laquelle q désigne zéro ou 1 ; et si q=0, Ri, identiques ou non, représentent un hydrogène, un groupement méthyle, hydroxyle, acétoxy, amino, mono- ou di-alkylamine éventuellement interrompus par un ou plusieurs atomes d'azote et/ou éventuellement substitués par un ou plusieurs groupes amine, hydroxyle, carboxyle, alkylthio éventuellement porteur d'un groupe amino, sulfonique ; ou si q=1, Ri, identiques ou non, représentent un hydrogène, ainsi que les sels formés par ces composés avec des bases ou des acides.
**(6)** Les polymères dérivés de la N-carboxyalkylation du chitosane comme le N-carboxyméthyl chitosane ou le N-carboxybutyl chitosane (Evalsan de Jan Dekker).
**(7)** Les polymères tels que décrits dans FR 1 400 366 : 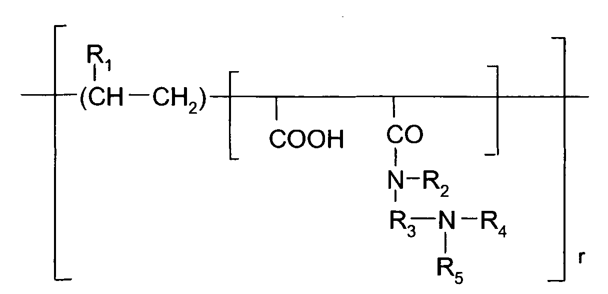 dans laquelle R₁ est un hydrogène, CH₃O- CH₃CH₂O-, phényle, R₂, R₅, identiques ou non, représentent un hydrogène, un radical alkyle (méthyle, éthyle), R₄ représente un radical alkyle (méthyle, éthyle) ou un radical de formule -R₃-N(R₅)₂, R₃ représentant -(CH₂)₂-, -(CH₂)₃-, -CH₂-CH(CH₃)-, ainsi que les homologues supérieurs de ces radicaux et contenant jusqu'à 6 atomes de carbone, r est tel que le poids moléculaire est compris entre 500 et 6000000 et de préférence entre 1000 et 1000000.
**(8)** Les polymères amphotères du type -D-X-D-X- choisis parmi:
   a) Les polymères obtenus par action de l'acide chloracétique ou le chloracétate de sodium sur les composés comportant au moins un motif de formule -D-X-D-X-D- où D désigne un radical -N[CH2CH2]2N- (pipérazinyle) et X désigne le symbole E ou E', E ou E' identiques ou différents désignent un radical bivalent qui est un radical alkylène à chaîne droite ou ramifiée comportant jusqu'à 7 atomes de carbone dans la chaîne principale substituée ou non par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule -D-X-D-X- où D désigne un radical -N[CH₂CH₂]₂N-(pipérazinyle) et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
**(9)** Les copolymères alkyl(C₁-C₅)-vinyléther / anhydride maléique modifié partiellement par semi-amidification avec une N,N-dialkylaminoalkylamine telle que la N,N-diméthyl-amino-propylamine ou par semi-estérification avec une N,N-dialcanolamine. Ces copolymères peuvent également comporter d'autres comonomères vinyliques tels que le vinylcaprolactame.

Parmi tous les polymères cationiques ou amphotères utilisables selon la présente invention, on préfère notamment :
(i) parmi les cationiques :
   - l'homopolymère de chlorure de diméthyldiallylammonium (Merquat 100DRY de Nalco);
   - les copolymères de chlorure de diméthyldiallylammonium et d'acrylamide (Merquat 2200 de Nalco);
   - les polymères de type poly(ammonium quaternaire) préparés et décrits dans FR 2 270 846, constitués de motifs récurrents de formules (W) et (U) suivantes : 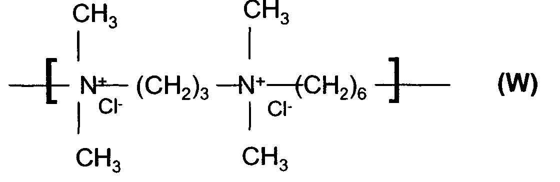 et notamment ceux dont le poids moléculaire, déterminé par chromatographie par perméation de gel, est compris entre 9500 et 9900; 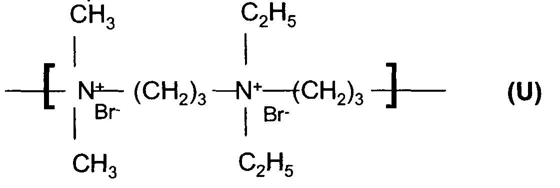 et notamment ceux dont le poids moléculaire, déterminé par chromatographie par perméation de gel, est d'environ 1200;
   - les polymères de type poly(ammonium quaternaire) de la famille (11) avec X- désignant le Chlore, et notamment ceux dont la masse moléculaire moyenne en poids est inférieure à 100 000, de préférence inférieure ou égale à 50 000;
(ii) parmi les polymères amphotères :
   - le copolymère chlorure de diméthyldiallylammonium/acide acrylique (80/20) (Merquat 280 DRY de Calgon- dénomination CTFA : Polyquaternium 22) ;
   - le copolymère chlorure de diméthyldiallylammonium/acide acrylique (95/5) (Merquat 295 DRY de Calgon) ;
   - le copolymère de chlorure de méthacrylamidopropyltrimonium, d'acide acrylique et d'acrylate d'éthyle (Merquat 2001 de Calgon - dénomination CTFA : Polyquaternium 47) ;
   - le terpolymère acrylamide/chlorure de diméthyldiallylammonium/acide acrylique (Merquat Plus 3330 DRY de Calgon -dénomination CTFA : Polyquaternium 39).

### Autres additifs

La composition de décoloration anhydre peut de même comprendre au moins une charge minérale, comme les argiles, les silices telles les silices pyrogénées à caractère hydrophile ou hydrophobe.
Elle peut de plus comprendre au moins un liant tel que la vinylpyrrolidone, au moins un lubrifiant comme les stéarates de polyol ou les stéarates de métaux alcalins ou alcalino-terreux, ainsi que des agents de contrôle du dégagement d'oxygène tels que le carbonate ou l'oxyde de magnésium.
La composition de décoloration anhydre peut comprendre le cas échéant un ou plusieurs agents choisis parmi les colorants, les agents matifiants comme les oxydes de titane, les agents séquestrants, les vitamines ou provitamines, les filtres solaires, les silicones, les parfums.

Bien entendu, l'homme de l'art veillera à choisir le ou les éventuels composés complémentaires mentionnés ci-avant, de manière telle que les propriétés avantageuses attachées intrinsèquement à la composition de décoloration et/ou à la composition de décoloration prête à l'emploi ne soient pas, ou substantiellement pas, altérées par la ou les adjonctions envisagées.

La composition de décoloration anhydre, sous forme de pâte, peut être préparée de manière classique, en dispersant, sous agitation mécanique, l'ensemble des composés pulvérulents dans le liquide inerte, dans lequel on a préalablement dispersé ou mélangé les autres composés liquides de la composition de décoloration.
On peut également préparer la pâte par extrusion, en introduisant les phases liquides et solides de la composition dans l'extrudeur, puis en les mélangeant à une température inférieure à 25°C à l'aide d'un système bi-vis co-rotatives composé d'éléments de transport et de malaxage.

La composition oxydante va maintenant être décrite.

### Composition oxydante

Cette dernière est une émulsion huile dans eau de peroxyde d'hydrogène, comprenant au moins un tensioactif non ionique et/ou anionique et au moins un copolymère obtenu à partir d'au moins un monomère à insaturation éthylénique à groupement sulfonique, sous forme libre ou partiellement ou totalement neutralisée et comprenant au moins une partie hydrophobe.

### Tensioactifs

En ce qui concerne les tensioactifs non ioniques et anioniques, on pourra se référer aux listes détaillées précédemment dans le cadre de la description des éléments constitutifs de la composition de décoloration anhydre.

De préférence, les alkylsulfates, les alkyléthersulfates de métal alcalin comme le sodium, le potassium, alcalino-terreux comme le magnésium, d'ammonium, d'amines, d'aminoalcools.

Il est de même avantageux de mettre en oeuvre au moins un tensioactif non ionique choisi parmi, seuls ou en mélanges, les alcools gras (notamment en C6-C24) polyéthoxylés et/ou polypropoxylés, le nombre de groupements oxyde d'éthylène et/ou oxyde de propylène étant habituellement compris entre 1 et 50 ; les alcools gras (notamment en C6-C24) mono- ou poly-glycérolés comportant en moyenne 1 à 30 groupements glycérol et les amides gras (notamment en C6-C24) polyglycérolés comportant en moyenne 1 à 5 groupements glycérol et en particulier 1,5 à 4.

Il est à noter que la teneur en tensioactifs présents dans la composition oxydante est en général comprise entre 0,05 et 30 % en poids, de préférence entre 0,1 et 20 % en poids.
Avantageusement, la composition prête à l'emploi, c'est-à-dire la composition de décoloration anhydre et la composition oxydante, présente une teneur totale en tensioactifs comprise entre 0,05 et 30 %, et de préférence entre 0,1 et 20 % en poids de la composition prête à l'emploi.

### Copolymère

Le copolymère présent dans la composition oxydante comprend au moins un motif provenant d'un monomère à insaturation éthylénique à groupement sulfonique, sous forme libre ou partiellement ou totalement neutralisée et comprenant au moins une partie hydrophobe.

Les monomères à insaturation éthylénique à groupement sulfonique sont choisis notamment parmi l'acide vinylsulfonique, l'acide styrène-sulfonique, les acides (méth)acrylamido(C₁-C₂₂)alkylsulfoniques, les acides N-(C₁-C₂₂)alkyl(méth)acrylamido(C₁-C₂₂)alkylsulfoniques comme l'acide undécyl-acrylamido-méthane-sulfonique ainsi que leurs formes partiellement ou totalement neutralisées.

Plus préférentiellement, on utilisera les acides (méth)acrylamido(C₁-C₂₂) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.
Plus particulièrement, on utilisera l'acide 2-acrylamido-2-méthylpropane-sulfonique (AMPS) ainsi que ses formes partiellement ou totalement neutralisées.

La neutralisation totale ou partielle des fonctions sulfoniques du copolymère peut être faite au moyen d'une base minérale, telle que la soude, la potasse, ammoniaque ; ou d'une base organique telle que la mono-, di- ou tri-éthanolamine, un aminométhyl-propanediol, la N-méthyl-glucamine, les acides aminés basiques comme l'arginine et la lysine, et les mélanges de ces composés.

La partie hydrophobe du copolymère comporte plus particulièrement 6 à 50 atomes de carbone, de préférence 6 à 22 atomes de carbone, et de manière encore plus avantageuse 6 à 18 atomes de carbone, et encore plus particulièrement 12 à 18 atomes de carbone.

En outre, les copolymères mis en oeuvre dans l'invention peuvent être réticulés ou non-réticulés. De préférence, on met en oeuvre des copolymères réticulés.
Les agents de réticulation peuvent être choisis parmi les composés à polyinsaturation oléfinique couramment utilisés pour la réticulation des polymères obtenus par polymérisation radicalaire. On peut citer par exemple, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.
On utilise plus particulièrement le méthylène-bis-acrylamide, le méthacrylate d'allyle ou le triméthylol propane triacrylate (TMPTA).

Le taux de réticulation varie en général de 0,01 à 10% en mole et plus particulièrement de 0,2 à 2% en mole par rapport au copolymère.

Les copolymères conformes à l'invention ont généralement un poids moléculaire moyen en nombre allant de 103 à 2.10⁷ g/mole, de préférence allant de 2.10³ à 5.10⁶ et plus préférentiellement encore de 105 à 15.105 g/mole.

Les copolymères conformes à l'invention peuvent plus particulièrement être choisis parmi les polymères amphiphiles statistiques d'AMPS modifiés par réaction avec une n-monoalkylamine ou une di-n-alkylamine en C₆-C₂₂, et tels que ceux décrits dans la demande de brevet WO00/31154, dont la description des polymères et de leur synthèse fait partie de la description. Rappelons que les termes polymère amphiphile signifient que ledit polymère comporte à la fois une partie hydrophile et une partie hydrophobe, et notamment une chaîne hydrophobe.

Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Les polymères préférés de l'invention sont choisis parmi les copolymères amphiphiles d'AMPS et d'au moins un monomère hydrophobe à insaturation éthylénique comportant au moins une partie hydrophobe ayant de 6 à 50 atomes de carbone et plus préférentiellement de 6 à 22 atomes de carbone et encore plus préférentiellement de 6 à 18 atomes de carbone et plus particulièrement 12 à 18 atomes de carbone.

Ces mêmes copolymères peuvent contenir en outre un ou plusieurs monomères éthyléniquement insaturés ne comportant pas de chaîne hydrophobe tels que les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans EP-A-750899, US 5089578, FR 2 818 543 et dans les publications de Yotaro Morishima suivantes :
- « Self-assembling amphiphilic polyelectrolytes and their nanostructures - Chinese Journal of Polymer Science Vol. 18, N°40, (2000), 323-336. »
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior - Langmuir, 2000, Vol.16, N°12, 5324-5332 » ;
- « Stimuli responsive amphiphilic copolymers of sodium 2-(acrylamido)-2-methylpropanesulfonate and associative macromonomers - Polym. Preprint, Div. Polym. Chem. 1999, 40(2), 220-221».

Les monomères hydrophobes à insaturation éthylénique de ces copolymères particuliers sont choisis de préférence parmi les acrylates ou les acrylamides de formule (I) suivante : 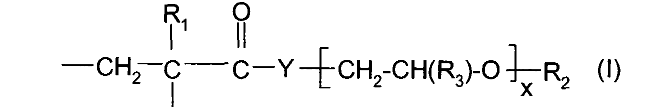 dans laquelle R₁ et R₃, identiques ou différents, désignent un hydrogène ou un radical alkyle linéaire ou ramifié en C₁-C₆ (de préférence méthyle) ; Y désigne O ou NH ; R₂ désigne un radical hydrocarboné hydrophobe en C₆-C₅₀, de préférence en C₆-C₂₂, plus particulièrement en C₆-C₁₈, encore plus préférentiellement en C₁₂-C₁₈ atomes de carbone ; x entier varie de 0 à 100.

Le radical R₂ est choisi de préférence parmi les radicaux alkyles en C₆-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)) ; les radicaux alkylperfluorés en C₆-C₁₈ (par exemple le groupement de formule -(CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulièrement préférée de l'invention, le monomère de formule (I) comporte au moins un motif oxyde d'alkylène (x ≥ 1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie plus spécialement entre 3 et 100 et plus particulièrement entre 3 et 50 et encore de préférence entre 7 à 25.

Parmi ces copolymères, on peut citer :
- les copolymères réticulés ou non, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au copolymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, tels que ceux décrits dans le brevet US- 5089578 ;
- les copolymères réticulés ou non d'AMPS partiellement ou totalement neutralisés et de méthacrylate de dodécyle ou de n-dodécylméthacrylamide, tels que ceux décrits dans les articles de Morishima cités ci-dessus.

On citera plus particulièrement les copolymères constitués de motifs acide 2-acrylamido-2-méthylpropane-sulfonique (AMPS) de formule (II) suivante : 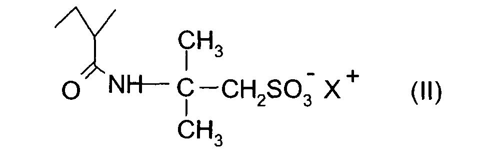 dans laquelle X⁺ est un proton, un cation de métal alcalin, un cation alcalino-terreux ou l'ion ammonium,
et de motifs de formule (III) suivante : 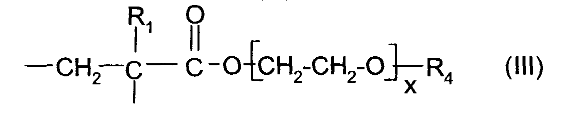 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₆-C₂₂ et plus préférentiellement en C₁₀-C₂₂.

Les polymères particulièrement préférés sont ceux pour lesquels x = 25 , R₁ désigne méthyle et R₄ représente n-dodécyle ; ils sont décrits dans les articles de Morishima mentionnés ci-dessus.
Les polymères pour lesquels X⁺ désigne sodium ou ammonium sont plus particulièrement préférés.

La concentration molaire en % des motifs de formule (II) et des motifs de formule (III) dans les copolymères varie en fonction de l'application cosmétique souhaitée et des propriétés rhéologiques de la formulation recherchées. De préférence pour les polymères les plus hydrophobes, la proportion molaire en motifs de formule (I) ou (III) varie de 50,1 à 99,9%, plus particulièrement de 70 à 95% et encore plus particulièrement de 80 à 90%.
De préférence pour les polymères peu hydrophobes, la proportion molaire en motifs de formule (I) ou (III) varie de 0,1 à 50%, plus particulièrement de 5 à 25% et encore plus particulièrement de 10 à 20%.

La distribution des monomères dans les polymères de l'invention peut être, par exemple, alternée, bloc (y compris multibloc) ou quelconque.

Ces copolymères amphiphiles peuvent être obtenus selon les procédés classiques de polymérisation radicalaire en présence d'un ou plusieurs initiateurs tels que par exemple, l'azobisisobutyronitrile (AIBN), l'azobisdiméthyl valéronitrile, le ABAH (le chlorhydrate de 2,2-azobis-[2-amidinopropane]), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.
Ils sont notamment obtenus par polymérisation radicalaire en milieu tert-butanol dans lequel ils précipitent.
La réaction peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.

Selon ce procédé, on a notamment polymérisé l'acide 2-acrylamido-2-méthylpropane-sulfonique ou l'un de ses sels de sodium ou d'ammonium, avec un ester de l'acide (méth)acrylique et d'un alcool en C₁₀-C₁₈ comprenant 8 motifs oxyde d'éthylène (8 OE) (Genapol®C-080 de Hoechst/Clariant) ; d'un alcool oxo en C₁₁ 8 OE (Genapol® UD-080) ; d'un alcool oxo en C₁₁ 7 OE (Genapol® UD-070) ; d'un alcool en C₁₂-C₁₄ 7 OE (Genapol® LA-070) ; d'un alcool en C₁₂-C₁₄ 9 OE (Genapol® LA-090) ; d'un alcool en C₁₂-C₁₄ 11 OE (Genapol® LA-110) ; d'un alcool en C₁₆-C₁₈ 8 OE (Genapol® T-080) ; d'un alcool en C₁₆-C₁₈ 15 OE (Genapol® T-150) ; d'un alcool en C₁₆-C₁₈ 11 OE (Genapol® T-110) ; d'un alcool en C₁₆-C₁₈ 20 OE (Genapol® T-200) ; d'un alcool en C₁₆-C₁₈ 25 OE (Genapol® T-250) ; d'un alcool en C₁₈- C₂₂ 25 OE et/ou d'un alcool iso C₁₆-C₁₈ 25 OE.

Les viscosités (mesurées à 25°C au viscosimètre Brookfield aiguille 7) des solutions aqueuses à 1% vont de préférence de 20 000 mPas à 100 000 mPas et plus particulièrement de 60 000 mPas à 70 000 mPas.

Selon un mode de réalisation particulier de l'invention, la teneur en copolymère représente 0,005 à 15 % en poids de la composition prête à l'emploi, plus particulièrement de 0,05 à 7,5 % en poids de la composition prête à l'emploi, de préférence de 0,1 à 5 % en poids de la composition prête à l'emploi.

### Phase huile de l'émulsion

La phase huile de l'émulsion comprend de préférence au moins un alcool gras.

Par alcool gras selon l'invention, on entend tout alcool gras, saturé ou insaturé, linéaire ou ramifié. Parmi ces alcools gras on préfère ceux en C₁₂₋C₂₂.
On peut citer plus particulièrement parmi eux, les alcools laurique, cétylique, stéarylique, oléïque, béhénique, linoléique, undécylénique, palmitoléïque, linolénique, arachidonique, érucique, et leurs mélanges. L'alcool cétylique est plus particulièrement préféré.

Dans les émulsions oxydantes selon l'invention, la concentration en alcools gras peut varier d'environ 0,1 à 30% en poids et plus préférentiellement d'environ 0,5 à 15% en poids par rapport au poids total de l'émulsion.

### Additifs

La composition oxydante peut de même comprendre les additifs usuels dans le domaine, comme par exemple des agents séquestrants tels l'éthylènediaminetétraacétique, le pentasodium pentetate (dénomination CTFA) ou l'acide étidronique ; des agents stabilisants l'eau oxygénée tels les sels de stannate et de pyrophosphate de métaux alcalins (comme le sodium, le potassium par exemple), ou le salicylate de sodium ; des colorants ; des parfums ; des agents anti-mousse ; des polymères substantifs cationiques ou amphotères tels que ceux décrits plus haut.

Bien entendu, l'homme de l'art veillera à choisir le ou les éventuels composés complémentaires mentionnés ci-avant, afin que les propriétés de la composition de décoloration prête à l'emploi ne soient pas, ou substantiellement pas, altérées par la ou les adjonctions envisagées.

La teneur en peroxyde d'hydrogène dans la composition oxydante est avantageusement comprise entre 1 et 12 % en titre en peroxyde d'hydrogène, plus particulièrement comprise entre 2 et 12 % en titre en peroxyde d'hydrogène, et de préférence entre 2,7 et 12 % en titre en peroxyde d'hydrogène.

La teneur en peroxyde d'hydrogène dans la composition de décoloration prête à l'emploi est, selon un mode de réalisation particulier de l'invention, comprise entre 1 et 12 % en titre en peroxyde d'hydrogène, de préférence de 2 à 9 % en titre en peroxyde d'hydrogène, et plus préférentiellement de 2 à 6 % en titre en peroxyde d'hydrogène.

Plus particulièrement, le pH de la composition oxydante est compris entre 1 et 6, et préférentiellement entre 2 et 4.
Le pH acide garantit la stabilité du peroxyde d'hydrogène dans la composition oxydante.
Il peut être obtenu à l'aide d'agents acidifiants tels que par exemple l'acide chlorhydrique, l'acide acétique, l'acide phosphorique, l'acide lactique, l'acide citrique, l'acide salicylique ou l'acide borique.
En outre, le pH peut être ajusté classiquement et si nécessaire par ajout d'agents basifiants, tels que par exemple, l'ammoniaque, la monoéthanolamine, la diéthanolamine, la triéthanolamine, l'isopropanolamine, le 1,3-diamino-propane, un (bi)carbonate alcalin ou d'ammonium, un carbonate organique tel que le carbonate de guanidine, ou bien encore un hydroxyde alcalin, tous ces composés pouvant bien entendu être pris seuls ou en mélange.

La composition oxydante est préparée en mélangeant à température ambiante le peroxyde d'hydrogène et les autres ingrédients de la phase aqueuse de l'émulsion huile dans eau puis de réaliser l'émulsion par ajout de la phase huile de l'émulsion, à une température supérieure à la température ambiante.

Le procédé de préparation de la composition de décoloration prête à l'emploi selon l'invention consiste à mélanger la composition de décoloration anhydre et la composition oxydante. Ce mélange doit se faire immédiatement avant l'application du produit sur les fibres à décolorer.

Habituellement, on mélange la composition de décoloration avec environ 0,5 à 10 équivalents en poids de la composition oxydante.

Notons que le pH de la composition prête à l'emploi selon l'invention est, avantageusement compris entre 4 et 12, plus particulièrement entre 7 et 11,5, et de préférence entre 8 et 11.

La présente invention a de même pour objet un procédé de décoloration de fibres kératiniques humaines, en particulier des cheveux, consistant à appliquer sur la zone des fibres kératiniques humaines, sèches ou humides, à décolorer, la composition de décoloration prête à l'emploi selon l'invention ; à la laisser agir pendant un temps de pause suffisant pour obtenir la décoloration recherchée ; à éliminer la composition par un rinçage à l'eau, suivi d'un lavage avec un shampooing, puis éventuellement d'un séchage.

Habituellement, le temps de pause varie entre 1 et 60 minutes, plus préférentiellement entre 10 et 50 minutes.

L'invention a enfin pour objet un dispositif à plusieurs compartiments, ou "kit", pour la mise en oeuvre du procédé de décoloration des fibres kératiniques humaines.
Ce dispositif comporte au moins deux compartiments dont l'un contient une composition de décoloration anhydre sous forme de pâte comprenant au moins un sel peroxygéné, au moins un agent alcalin et de 15 à 35 % en poids d'au moins un liquide inerte organique ; et l'autre contient une composition oxydante sous la forme d'une émulsion huile dans eau de peroxyde d'hydrogène, comprenant au moins un tensioactif non ionique et/ou anionique et au moins un copolymère comprenant au moins un motif provenant d'un monomère à insaturation éthylénique à groupement sulfonique, sous forme libre ou partiellement ou totalement neutralisée et comprenant au moins une partie hydrophobe.

Des exemples non limitatifs de la présente invention vont maintenant être présentés.

### Exemples

### Composition de décoloration A sous forme de pâte anhydre

On prépare la composition A en mélangeant les composés suivants :

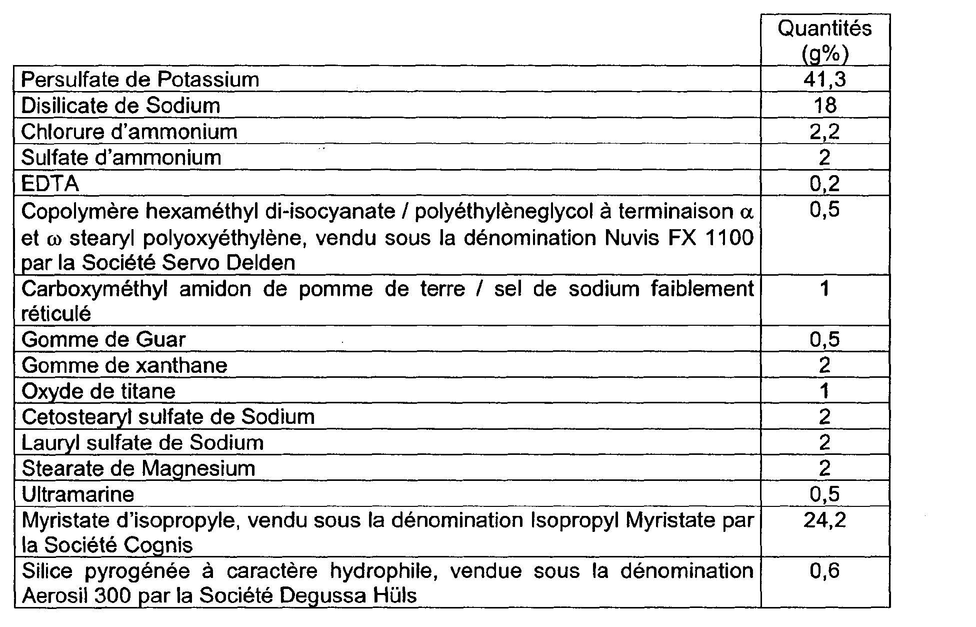

### Compositions oxydantes (Emulsions H/E)

On prépare l'émulsion par mélange des composés suivants :

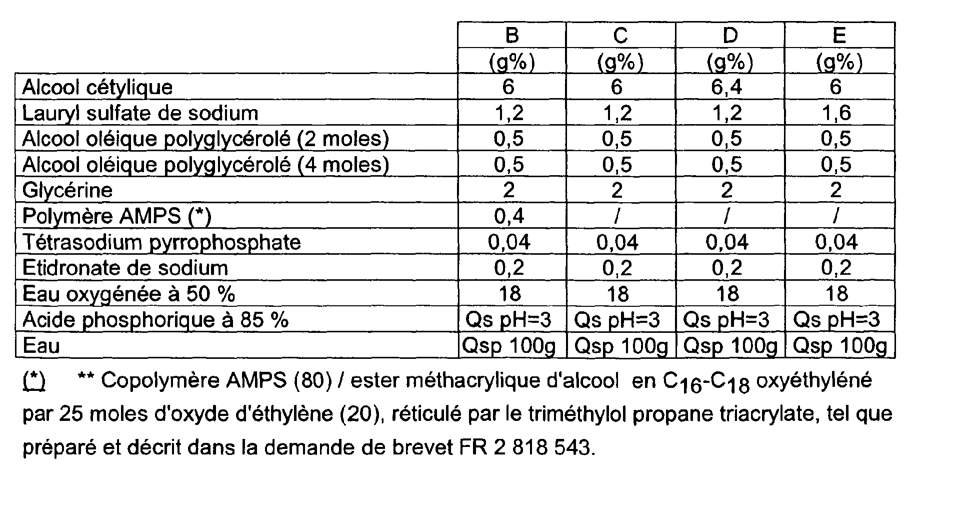

### Compositions aqueuses de décoloration prêtes à l'emploi

- COMP A/B : 20g de composition de décoloration A + 30g de composition oxydante B
- COMP A/C : 20g de composition de décoloration A + 30g de composition oxydante C
- COMP A/D : 20g de composition de décoloration A + 30g de composition oxydante D
- COMP A/E : 20g de composition de décoloration A + 30g de composition oxydante E

Les compositions sont obtenues par mélange.

### Evaluation de la rapidité de mélange des compositions aqueuses de décoloration prêtes à l'emploi :

- Evaluation du temps de mélange en secondes (cf. via un chronomètre)
- Déclenchement du chronomètre au premier coup de spatule
- Arrêt du chronomètre lorsque les mélanges sont lisses et homogènes
- Evaluation par un panel de 5 personnes

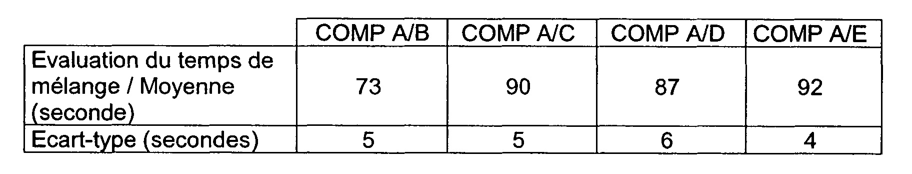

Le mélange des compositions A et B (COMP A/B) selon l'invention est significativement plus rapide à réaliser.

De plus, la composition de décoloration prête à l'emploi COMP A/B s'applique facilement et rapidement. Elle présente une très bonne adhérence. Elle ne coule pas hors des zones de chevelure que l'on souhaite décolorer. Elle permet enfin une décoloration puissante et homogène, tout en offrant de très bonnes propriétés cosmétiques.

## Revendications

1. Composition de décoloration de fibres kératiniques humaines, et en particulier des cheveux, prête à l'emploi, susceptible d'être obtenue en mélangeant avant emploi :
i) une composition de décoloration anhydre sous forme de pâte comprenant au moins un sel peroxygéné, au moins un agent alcalin et de 15 à 35 % en poids d'au moins un liquide inerte organique, avec,
ii) une composition oxydante sous forme d'une émulsion huile dans eau de peroxyde d'hydrogène, comprenant au moins un tensioactif non ionique et/ou anionique et au moins un copolymère comprenant au moins un motif provenant d'un monomère à insaturation éthylénique à groupement sulfonique, sous forme libre ou partiellement ou totalement neutralisée et comprenant au moins une partie hydrophobe.

2. Composition selon la revendication précédente, **caractérisée en ce que** le liquide inerte organique de la composition de décoloration anhydre, comprend au moins un composé choisi parmi les polydécènes, les mono- et poly- esters d'acides carboxyliques, les mono- ou poly- esters de sucres d'acides en C8-C30, les éthers cycliques, les esters cycliques, les huiles silicones, les huiles minérales, les huiles végétales, ou leurs mélanges.

3. Composition selon l'une quelconque des revendications précédentes, caractérisée en que le liquide inerte est choisi parmi les esters d'acides en C8-C30 et d'un monoalcool saturé, linéaire ou ramifié, en C3-C6.

4. Composition selon l'une quelconque des revendications précédentes, caractérisée en que le sel peroxygéné présent dans la composition de décoloration anhydre est choisi parmi les persulfates, perborates, percarbonates et peroxydes de métaux alcalins et alcalino-terreux, ou leurs mélanges.

5. Composition selon la revendication précédente, caractérisée en que le sel peroxygéné est choisi parmi les persulfates de sodium et de potassium, seuls ou en mélange.

6. Composition selon l'une quelconque des revendications précédentes, **caractérisée en ce que** la teneur en sel peroxygéné dans la composition de décoloration anhydre est comprise entre 10 et 70 % en poids, de préférence entre 20 et 60 % en poids.

7. Composition selon l'une quelconque des revendications précédentes, **caractérisée en ce que** la teneur en sel peroxygéné est comprise entre 5 et 35 % en poids de la composition prête à l'emploi, de préférence entre 10 et 30 % en poids de la composition prête à l'emploi.

8. Composition selon l'une quelconque des revendications précédentes, **caractérisée en ce que** l'agent alcalin présent dans la composition de décoloration anhydre est choisi parmi l'urée ; les sels d'ammonium ; les silicates, les phosphates, les carbonates de métaux alcalins ou alcalino-terreux, seuls ou en mélange.

9. Composition selon l'une quelconque des revendications précédentes, **caractérisée en ce que** la teneur en agent alcalin dans la composition de décoloration anhydre est comprise entre 0,01 et 40 % en poids, de préférence entre 0,1 et 30 % en poids.

10. Composition selon l'une quelconque des revendications précédentes, **caractérisée en ce que** la teneur en agent alcalin est comprise entre 0,005 et 20 % en poids de la composition prête à l'emploi, de préférence entre 0,05 et 15 % en poids de la composition prête à l'emploi.

11. Composition selon l'une quelconque des revendications précédentes, **caractérisée en ce que** la composition de décoloration anhydre comprend au moins un tensioactif non ionique, anionique, amphotère, zwitterionique ou cationique, ou leurs mélanges.

12. Composition selon l'une quelconque des revendications précédentes, **caractérisée en ce que** la composition de décoloration anhydre comprend au moins un agent épaississant hydrosoluble dépourvu de chaîne hydrophobe.

13. Composition selon la revendication 12, **caractérisée en ce que** la teneur en agent épaississant dans la composition de décoloration anhydre représente 0,01 à 30 % en poids de la composition prête à l'emploi.

14. Composition selon l'une quelconque des revendications précédentes, **caractérisée en ce que** la composition de décoloration anhydre comprend au moins un polymère amphiphile comportant au moins une chaîne hydrophobe, de préférence différent de celui ou ceux présents dans l'émulsion huile dans eau.

15. Composition selon la revendication 14, **caractérisée en ce que** la teneur en polymère amphiphile comportant au moins une chaîne hydrophobe représente 0,01 à 30 % en poids de la composition prête à l'emploi.

16. Composition selon l'une quelconque des revendications précédentes, **caractérisée en ce que** la composition de décoloration anhydre comprend moins de 1 % en poids d'eau, et de préférence moins de 0,5 % en poids d'eau.

17. Composition selon l'une quelconque des revendications précédentes, **caractérisée en ce que** le copolymère de la composition oxydante comprend au moins un motif provenant d'un monomère à insaturation éthylénique à groupement sulfonique choisi parmi l'acide vinylsulfonique, l'acide styrène-sulfonique, les acides (méth)acrylamido(C₁-C₂₂)alkylsulfoniques, les acides N-(C₁-C₂₂)alkyl(méth)acrylamido(C₁-C₂₂)alkylsulfoniques comme l'acide undécyl-acrylamido-méthane-sulfonique ainsi que leurs formes partiellement ou totalement neutralisées.

18. Composition selon la revendication précédente, **caractérisée en ce que** le copolymère comprend au moins une partie hydrophobe comprenant 6 à 50 atomes de carbone, plus particulièrement de 6 à 22 atomes de carbone, de préférence de 6 à 18 atomes de carbone, et encore plus préférentiellement de 12 à 18 atomes de carbone.

19. Composition selon l'une quelconque des revendications précédentes, **caractérisée en ce que** la teneur en copolymère représente 0,005 à 15 % en poids de la composition prête à l'emploi, plus particulièrement de 0,05 à 7,5 % en poids de la composition prête à l'emploi, de préférence de 0,1 à 5 % en poids de la composition prête à l'emploi.

20. Composition selon l'une quelconque des revendications précédentes, **caractérisée en ce que** l'émulsion comprend :
au moins un tensioactif anionique choisi parmi, seuls ou mélanges, les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfo-succinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates ; les esters d'alkyl(C₆-C₂₄)polyglycosides carboxyliques ; les acyliséthionates, les N-acyltaurates ; les sels d'acides gras ; les acides d'alkyl D galactoside uroniques et leurs sels ; les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels.
et/ou au moins un tensioactif non ionique choisi parmi, seuls ou en mélange, les alcools, les alpha-diols, les alkylphénols polyéthoxylés et/ou polypropoxylés, ayant une chaîne comportant par exemple 8 à 22 atomes de carbone ; les copolymères d'oxyde d'éthylène et de propylène ; les condensats d'oxyde d'éthylène et de propylène sur des alcools gras ; les amides gras polyéthoxylés ; les alcools gras mono- ou poly-glycérolés; les amides gras polyglycérolés ; les esters d'acides gras du sorbitan oxyéthylénés ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.

21. Composition selon l'une quelconque des revendications 11 ou 20, **caractérisée en ce que** la teneur totale en tensioactif représente 0,05 et 30 % en poids de la composition prête à l'emploi, de préférence entre 0,1 et 20 % en poids de la composition prête à l'emploi.

22. Composition selon l'une quelconque des revendications précédentes, **caractérisée en ce que** la phase huile de l'émulsion huile dans eau comprend au moins un alcool gras.

23. Composition selon l'une quelconque des revendications précédentes, **caractérisée en ce que** la teneur en peroxyde d'hydrogène dans la composition prête à l'emploi représente de 1 à 12 % en titre en peroxyde d'hydrogène, plus particulièrement de 2 à 9% en titre en peroxyde d'hydrogène, de préférence de 2 à 6 % en titre en peroxyde d'hydrogène.

24. Composition selon l'une quelconque des revendications précédentes, **caractérisée en ce que** le pH de l'émulsion huile dans eau est compris entre 1 et 6,de préférence entre 1 et 4.

25. Composition selon l'une quelconque des revendications précédentes, **caractérisée en ce que** le pH de la composition prête à l'emploi est compris entre 4 et 12, plus particulièrement entre 7 et 11,5, de préférence entre 8 et 11.

26. Procédé de préparation de la composition de décoloration de fibres kératiniques humaines, et en particulier des cheveux, prête à l'emploi, selon l'une quelconque des revendications 1 à 25, **caractérisé en ce que** l'on mélange avant emploi :
i) une composition de décoloration anhydre sous forme de pâte comprenant au moins un sel peroxygéné, au moins un agent alcalin et de 15 à 35 % en poids d'au moins un liquide inerte organique, avec,
ii) une composition oxydante sous la forme d'une émulsion huile dans eau de peroxyde d'hydrogène, comprenant au moins un tensioactif non ionique et/ou anionique et au moins un copolymère comprenant au moins un motif provenant d'un monomère à insaturation éthylénique à groupement sulfonique, sous forme libre ou partiellement ou totalement neutralisée et comprenant au moins une partie hydrophobe.

27. Procédé de décoloration de fibres kératiniques humaines, en particulier des cheveux, consistant à appliquer la composition de décoloration prête à l'emploi selon l'une quelconque des revendications 1 à 25, sur la zone des fibres kératiniques humaines, sèches ou humides, à décolorer ; à la laisser agir pendant un temps de pause suffisant pour obtenir la décoloration recherchée ; à éliminer la composition par un rinçage à l'eau, suivi d'un lavage avec un shampooing, puis éventuellement d'un séchage.

28. Procédé de décoloration selon la revendication précédente, **caractérisé en ce que** le temps de pause varie entre 1 et 60 minutes, plus préférentiellement entre 10 et 50 minutes.

29. Dispositif à plusieurs compartiments, ou "kit", pour la mise en oeuvre du procédé selon l'une des revendications 26 ou 27, **caractérisé par le fait qu'**il comporte au moins deux compartiments dont l'un contient une composition de décoloration anhydre sous forme de pâte comprenant au moins un sel peroxygéné, au moins un agent alcalin et de 15 à 35 % en poids d'au moins un liquide inerte organique ; et l'autre contient une composition oxydante sous la forme d'une émulsion huile dans eau de peroxyde d'hydrogène, comprenant au moins un tensioactif non ionique et/ou anionique et au moins un copolymère comprenant au moins un motif provenant d'un monomère à insaturation éthylénique à groupement sulfonique, sous forme libre ou partiellement ou totalement neutralisée et comprenant au moins une partie hydrophobe.
